# EUROPÄISCHE PATENTSCHRIFT

(11) **EP 0 366 127 B1**
(45) Veröffentlichungstag und Bekanntmachung des Hinweises auf die Patenterteilung: **08.05.1996**
(21) Anmeldenummer: 89119860.8
(22) Anmeldetag: 25.10.1989
(51) Int. Cl.: A61B 5/04, A61B 5/03

(54) **Verwendung eines Katheters und Messvorrichtung zur Messung von Motilität und Peristaltik in schlauchförmigen, ihren Inhalt transportierenden Organen mittels simultaner multipler Impedanzmessung**
Use of a catheter and measuring device for measuring the motility and the peristaltic in tubular body parts by means of simultaneous measurement of a plurality of impedances
L'emploi d'un cathéter et dispositif de mesure destiné à la mesure de la motilité et du mouvement péristaltique des organes tubulaires par la mesure simultanée d'une pluralité d'impédances

(30) Priorität: 25.10.1988 DE 3836349
(43) Veröffentlichungstag der Anmeldung: 02.05.1990
(73) Patentinhaber: Forschungsgesellschaft für Biomedizinische Technik e.V., D-52074 Aachen (DE)
(72) Erfinder: Silny, Jiri, Dr.-Ing., D-5100 Aachen (DE); Rau, Günter, Prof. Dr. rer. nat., D-5100 Aachen (DE)
(74) Vertreter: TER MEER - MÜLLER - STEINMEISTER & PARTNER

(56) Entgegenhaltungen:
- DE-A- 3 327 561
- GB-A- 2 141 827
- US-A- 4 561 450
- US-A- 4 706 688
- US-A- 5 109 870
- PROCEEDINGS OF THE NINTH ANNUAL CONFERENCE OF THE IEEE ENGINEERING IN MEDICINE AND BIOLOGY SOCIETY, Boston, 13.-16. November 1987, Band 4, Seiten 1960-1961,IEEE, New York, US; D. PENG et al.: "Design of a self-adhering gastricmultichannel electrode assembly"

## Beschreibung

Die Erfindung betrifft die Verwendung eines Katheters in Form eines Schlauchs oder Strangs aus einem elektrisch nicht leitenden Kunststoff, der ähnlich wie in US-A-4,706,688 beschrieben, im Meßbereich eine Mehrzahl in definiertem gegenseitigem Abstand angeordnete, den Kunststoffschlauch oder Kunststoffstrang wenigstens teilweise ringförmig umgebende Elektroden trägt, deren Signalzuleitungen oder -ableitungen im Inneren des Schlauchs oder Strangs verlaufen bzw. in diesen eingebettet sind. Die Erfindung bezieht sich weiterhin auf eine Meßvorrichtung unter Verwendung eines solchen Katheters, mit der sich Signale zur Beurteilung von Motilität und/oder Peristaltik gewinnen lassen.

Zur Diagnostik von krankheitsbedingten Störungen der Motilität bzw. der Peristaltik stehen heute zwei grundsätzlich unterschiedliche Verfahrensmöglichkeiten zur Verfügung, nämlich
a) die Manometrie mittels der Perfusionsmeßtechnik oder mittels Halbleiterdruckaufnehmern und
b) bildgebende Verfahren, die sich entweder der röntgenologischen Darstellung bzw. Szintigraphie der mit Kontrastmitteln versehenen Inhalte bedienen.

### Zu a):

Bei der Manometrie wird eine Druckänderung zur Charakterisierung der Kontraktionsabläufe in einem schmalen Abschnitt des zu untersuchenden Organs herangezogen. Die größte Verbreitung hat bei dieser Art von Messung die Perfusionsmeßtechnik erfahren. Dabei wird die Druckänderung bestimmt, die sich im Katheter, der mit einem konstanten Flüssigkeitsstrom durchflossen wird, bei Behinderung des Ausflusses durch die Kontraktion eines betreffenden Organs ergibt, gemessen. Da hierbei aber pro Kanal Schläuche mit einem inneren Durchmesser von über 1 mm verwendet werden müssen, ist ein Multikatheter mit bis zu acht Kanälen hinsichtlich seines Durchmessers gerade noch zu akzeptieren. Ein weiterer wichtiger Nachteil ist aber, daß von einem Perfusionskatheter in die Organe Flüssigkeit eingeleitet wird, welche die Funktion des zu messenden Organs gerade bei einer Langzeitmessung wesentlich beeinträchtigen kann.

### Zu b):

Bildgebende Verfahren sind prinzipiell geeignet, den Nachweis der Motilität beispielsweise in der Speiseröhre zu erbringen. Die Diagnostik im Darmbereich dagegen gestaltet sich wegen der vielen Überlagerungen problematisch. Darüber hinaus muß der hohe Kostenaufwand für die Anschaffung, den Betrieb und nicht zuletzt auch für die Auswertung der gewonnenen Bilder in Betracht gezogen werden.

Der Erfindung liegt die Aufgabe zugrunde, ein Verfahren und eine relativ einfach zu realisierende Meßvorrichtung zur Beurteilung von Motilität und/oder Peristaltik in schlauchförmigen, ihren Inhalt transportierenden Organen zu schaffen, das sich universeller einsetzen läßt und zu verläßlichen Diagnoseergebnissen führt.

Die Erfindung sieht zur Gewinnung von Signalen zur Beurteilung von Motilität und/oder Peristaltik in schlauchförmigen, ihren Inhalt transportierenden Organen bzw. zur Bestimmung der dynamischen Nachgiebigkeit von schlauchförmigen Leitungsorganen mittels simultaner multipler Impedanzmessung erfindungsgemäß die Verwendung eines im Patentanspruch 1 definierten Katheters vor.

Vorteilhafte Weiterbildungen der erfindungsgemäßen Verwendung eines solchen Katheters sind in weiteren Patentansprüchen angegeben und unter anderem in der nachfolgenden Beschreibung erläutert.

Eine Meßeinrichtung zur Bestimmung von Motilität und/oder Peristaltik in schlauchförmigen, ihren Inhalt transportierenden Organen unter Verwendung eines derartigen Katheters ist Gegenstand des Patentanspruchs 7 und weiterer abhängiger Patentansprüche.

Die erfindungsgemäße Verwendung eines solchen Katheter erlaubt es, Signale und Daten zu gewinnen, die zur Beurteilung der Art der Motilität und Peristaltik räumlich und zeitlich dienen, nämlich von stationären, propulsiven, repulsiven Wellen, von Kontraktionsausbreitung usw. Das zugrunde liegende Meßverfahren kann zur Bestimmung von Charakteristika, wie z. B. der Passagezeit und Passagegeschwindigkeit, der Kontraktionsfrequenz und der Kontraktionshäufigkeit einzelner Organabschnitte herangezogen werden.

Der Katheter wird zur Aufnahme in ein Organ eingeführt und in einer definierten Lage gegen eine Längsverschiebung fixiert. Die zu verwendenden Signale werden durch eine **simultane oder quasi simultane Erfassung der Impedanz von mindestens drei bis vielen Kanälen** gewonnen, wobei typischerweise 32 und mehr Kanäle verwendet werden.

Vorteilhafte Ausführungsformen der Erfindung sowie weitere Einzelheiten derselben werden nachfolgend unter Bezug auf die Zeichnung in beispielsweiser Ausführungsform näher erläutert. Es zeigen:
- Fig. 1: die vergrößerte perspektivische Darstellung eines Meßbereichabschnitts eines schlauchförmigen Katheters;
- Fig. 2: eine vergrößerte Ausschnittdarstellung im Längsschnitt durch einen Meßbereich eines Katheters;
- Fig. 3: veranschaulicht ein Verfahren zur simultanen Registrierung der Speiseröhrenaktivität mit einem multiplen Meßkatheter bei erfindungsgemäßer Art Verwendung;
- Fig. 4: verdeutlicht auch in Verbindung mit Fig. 3 eine bestimmte Ausführungsform eines Katheters;
- Fig. 5: ein schematisches Blockschaltbild zur Verdeutlichung eines Meßaufbaus bei erfindungsgemäßer Verwendung eines Katheters; und
- Fig. 6: verschiedene Möglichkeiten zur schaltungsmäßigen Anordnung einzelner Meßkanäle.

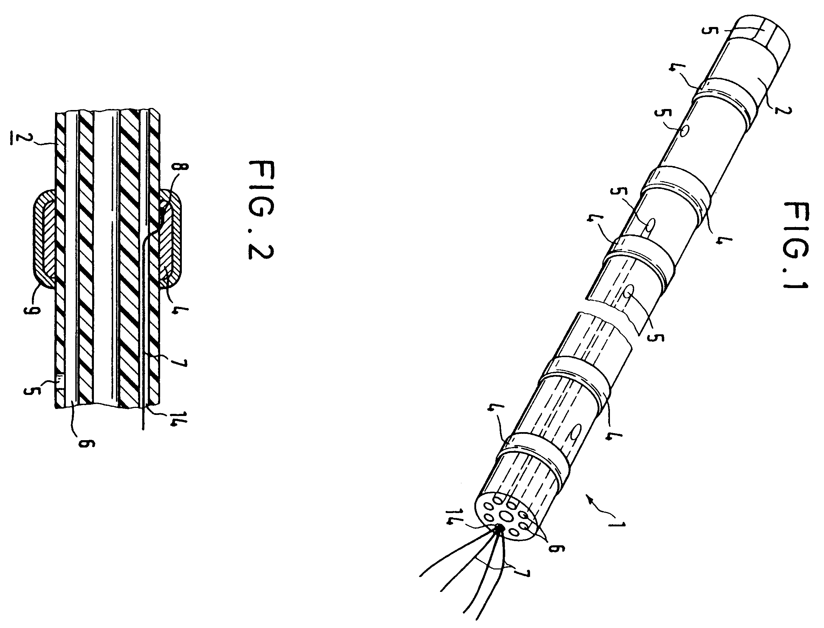

Die Fig. 1 läßt einen Teilabschnitt des Meßbereichs eines schlauchförmigen, vorzugsweise flexiblen Katheters 1 erkennen, der im wesentlichen aus einem Schlauch aus einem elektrisch nicht leitfähigen Kunststoff besteht. Als Materialien kommen hier z. B. Polyurethan, Polyamid, Polyethylen, Polytetrafluorethylen, Polyvinylchlorid oder auch bestimmte Siliconkautschukverbindungen in Frage.

Um die Außenseite des Schlauchs 2 sind in ungefähr gleichabständiger, jedoch nicht notwendigerweise gleichabständiger Folge ringförmige Elektroden 4 gelegt, die, wie die Fig. 2 erkennen läßt, aus einem flachen Bandmaterial hergestellt und mit dem Grundkörper des Katheters, also mit dem Schlauch 2, festhaftend verbunden sind. Die Kanten der Elektroden 4 sind abgerundet und so ausgebildet und in das Schlauchmaterial eingelassen, daß es zu keinen Verletzungen kommen kann. Als Materialien für die Elektroden kommen Metalle, Elektrodenmaterialien zweiter Art und leitfähige Kunststoffe in Frage. Wichtige Eigenschaften der Elektroden 4 sind deren Niederohmigkeit geringer Polarisationsspannung in der oberen Elektrodenschicht und ihre Langzeitstabilität. Da eine Elektrode 4 einen Übergang zu einem Elektrolyten bildet, muß sie, um diese geforderten Eigenschaften zu haben, mit einer Schicht 9, z. B. Silber-Silberchlorid, versehen sein. Der Abstand zwischen den Elektroden 4 kann konstant oder nach einem räumlichen Muster ausgebildet sein. Weitere Variablen im Bereich der Elektroden sind deren Breite und der gegenseitige Abstand eines Elektrodenpaars, das bei der Impedanzmessung einem Meßkanal zugeordnet wird. Die Breite der Elektroden 4 kann unterschiedlich ausgebildet sein; sie richtet sich nach dem Durchmesser des Schlauchs 2 und nach den zu messenden Anwendungen.

Die Elektrodenzuleitungen verlaufen im Inneren des Schlauchs 2 beispielsweise einem separaten Kanal 14, der durch Zwischenwände von weiteren, im Schlauchinneren verlaufenden Einzelkanälen 6 getrennt ist, deren Funktion später erläutert wird. An einer inneren Kontaktstelle 8 ist eine jeweilige Zuleitung 7 mit der zugeordneten Elektrode 4 verbunden.

Der Katheter 1 kann mit einem oder mehreren Lumen 5 versehen werden, durch die an unterschiedlichen Stellen aus dem Organ Stoffe für Analysezwecke entnommen oder Stoffe oder Flüssigkeiten als Kontrastmittel oder zur Funktionsstimulation eingegeben werden können. Eine solche funktionelle Stimulation kann jedoch nicht nur über eine Flüssigkeit, sondern auch elektrisch über die Meßelektroden 4 selbst erfolgen. Darüber hinaus können die Elektroden zur Aufnahme von elektrischen Biosignalen verwendet werden.

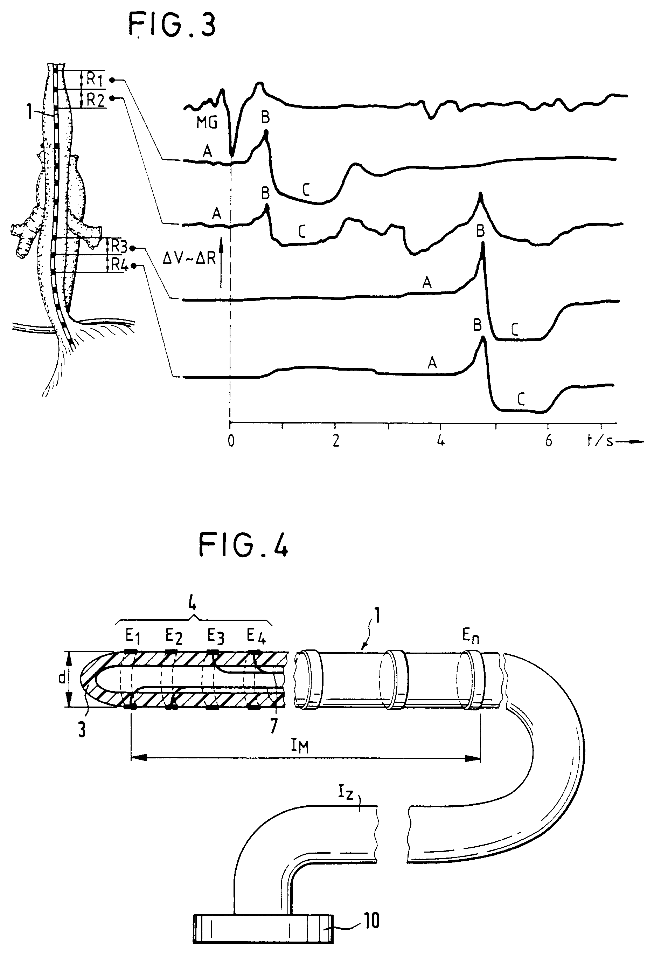

Die Prinzipdarstellung der Fig. 3 verdeutlicht die simultane Registrierung der Speiseröhrenaktivität mit einem multiplen Meßkatheter 1.

Ein Meßkanal wird dabei entweder zwischen zwei Elektroden 4 (E₁/E₂ bzw. E₂/E₃ bzw. E₃/E₄) zur Messung der Längsimpedanz oder zwischen einer Elektrode auf dem Katheter 1 und einer zentralen großflächigen Elektrode (nicht dargestellt) auf dem Körper ausgebildet.

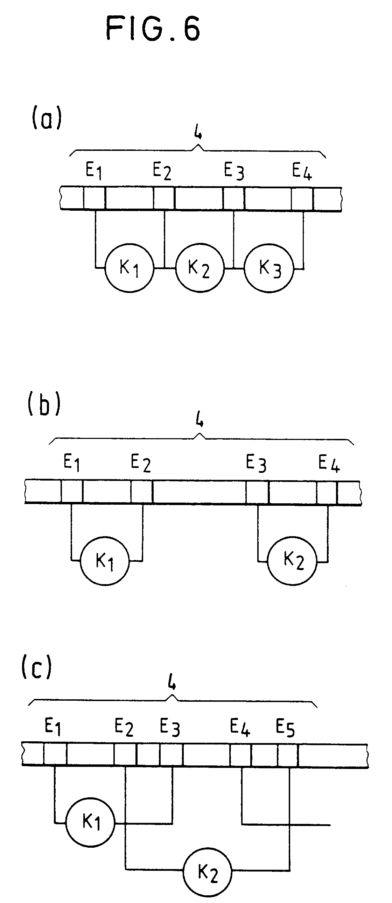

Die Längsimpedanz kann, wie die Fig. 6 verdeutlicht, entweder (a) sequentiell, (b) probenweise oder (c) mit Überlappung ermittelt werden.

In der Fig. 3 ist mit MG ein Referenzkanal bezeichnet, während die durch jeweilige Abstandspfeile markierten Größen R₁, R₂, R₃ und R₄ Impedanzveränderungen in den Kanälen 1, 2, 3 und 4 angeben. Im dargestellten Beispiel charakterisieren die Signale R₁ bis R₄ eine propulsive Kontraktionswelle. Aus den Laufzeiten zwischen zwei Kanälen kann die Passagezeit oder Passagegeschwindigkeit im jeweils untersuchten Organ errechnet werden.

Fig. 4 verdeutlicht weitere Einzelheiten einer abgewandelten Ausführungsform eines Katheters zur erfindungsgemäßen Verwendung. Die Darstellung läßt erkennen, daß der Katheterschlauch 2 am vorderen Ende 3 gut abgerundet verschlossen ist und mit seinen im Inneren verlaufenden Zuleitungen 7 an einen Stecker 10 angeschlossen ist. Der Durchmesser d kann bis zu wenigen mm betragen. Der Katheter ist flexibel, jedoch kann der Meßteil I_{M} des Katheters 1 unterschiedlich flexibel oder sogar starr ausgebildet sein kann, z.B. durch Verwendung von Keramikmaterialien. Die Länge des Meßteils I_{M} und der Zuleitungen I_{Z} können unterschiedlich mit bis zu einigen Metern Länge ausgebildet sein. Aufgrund des Schlauchinnenraums kann der Katheter vorübergehend, beispielsweise während der Messung, versteift werden.

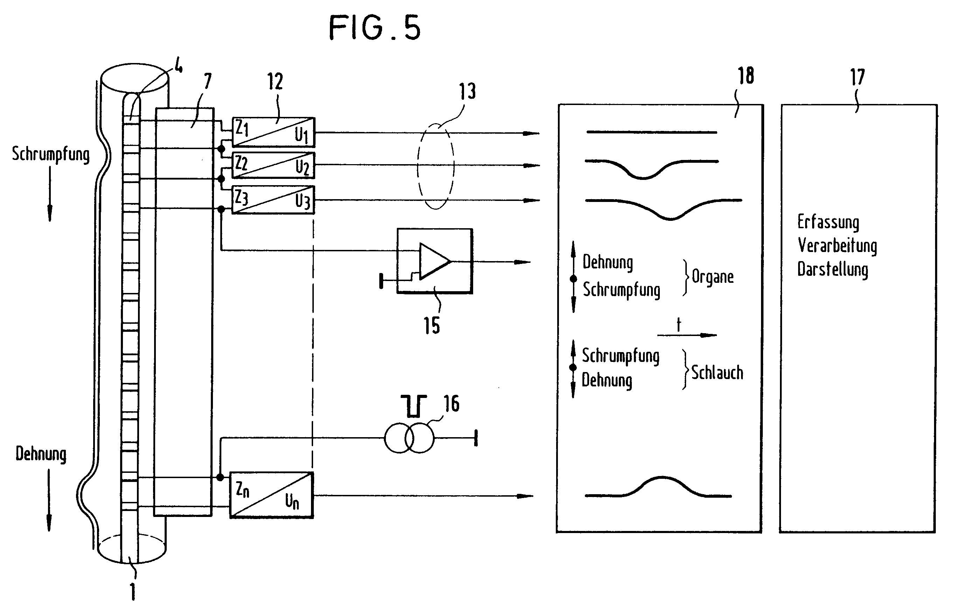

Der Katheter 1 ist mit wenigen (mindestens drei) bis zu vielen (z. B. 32 und mehr) Elektroden 4 (E₁, E₂, E₃, E₄, ...) versehen. Die Elektroden sind über die Zuleitungen 7 elektrisch leitend mit dem Stecker 10 einer Impedanzmeßanordnung 12 verbunden und auf eine Anzeige- bzw. Schreibeinrichtung 18 und/oder auf eine Signalverarbeitungseinheit 17 mit zugeordneter Anzeigevorrichtung geschaltet. Eine beispielsweise Ausführungsform des Anordnungsaufbaus ist in Fig. 5 veranschaulicht.

Außer mit den Lumen 5, deren Zweck und Funktion bereits erläutert wurde, kann der Katheter 1 mit einem oder mehreren Temperaturmeßfühlern (nicht dargestellt) entlang seiner Längsachse versehen sein.

Der Katheter 1 kann zusätzlich zur Impedanzmessung und gleichzeitig auch zur Aufnahme von EMG-Signalen herangezogen werden, die der mechanischen Kontraktion eines Organs voraneilen.

Über die Elektroden erlaubt der Katheter auch eine gleichzeitige funktionelle elektrische Stimulation oder Reizung des untersuchten Organs.

Darüber hinaus können in den Meßkatheter noch weitere Biosignal-Wandler 15 zur gleichzeitigen Aufnahme von elektrischen Biosignalen (z. B. EMG, EKG) mit kanalweise vorgesehenen Verstärkern zur Signaltrennung oder Stimulationseinrichtungen in einzelnen oder allen Kanälen, z. B. Stromquellen 16 zur elektrischen Stimulation oder Elemente für die Druckaufnahme oder zur Stimulation mit einem Druckballon integriert werden.

Bei erfindungsgemäßer Verwendung eines Meßkatheters der beschriebenen Art lassen sich die Motilitäts- und Peristaltikabläufe in gesunden wie auch erkrankten Organen, z. B. in der Speiseröhre, im Darm, im Urether, etc. erfassen. Die Fortbewegung der Inhalte dieser Organe Ist in der Regel in eine Richtung orientiert, obwohl z. B. im Darm auch zahlreiche Mischvorgänge vorkommen und bekannt sind, bei denen die Inhalte hin- und hertransportiert werden. Zur Fortbewegung der Inhalte müssen die Kontraktionsabläufe streng synchronisiert werden. Das Nachlassen bzw. ein totales Versagen der Spontanaktivität bereits eines kleinen Abschnitts kann zu Fehlfunktionen des gesamten Organs führen. Mit der hier vorgestellten erfindungsgemäßen Verwendung eines Katheters und der Meßeinrichtung lassen sich die Abschnitte des Organs mit einer fehlerhaften Funktion gut diagnostizieren.

Neben der Anwendung im medizinischen Bereich ist auch eine technische Anwendung möglich, so z. B. zur Messung der dynamischen Nachgiebigkeit von Schlauchwänden bei Applikation einer Impuls-Druckwelle zur Ermittlung der dynamischen Belastung der Wände. Damit lassen sich altersbedingte Veränderungen einer Schlauchwand rechtzeitig erkennen und beseitigen.

## Patentansprüche

1. Verwendung eines Katheters in Form eines Schlauchs (2) oder Strangs aus einem elektrisch nicht leitenden Kunststoff, der im Meßbereich eine Mehrzahl in definiertem gegenseitigen Abstand angeordnete, den Kunststoffschlauch (2) oder Kunststoffstrang wenigstens teilweise ringförmig umgebende Elektroden (4) trägt, deren Signalzuleitungen oder Ableitungen im Inneren des Schlauchs (2) oder Strangs verlaufen zur Gewinnung von Signalen zur Beurteilung von Motilität und/oder Peristaltik in schlauchförmigen, ihren Inhalt transportierenden Organen bzw. zur Bestimmung der dynamischen Nachgiebigkeit von schlauchförmigen Leitungsorganen mittels simultaner multipler Impedanzmessung.

2. Verwendung nach Anspruch 1, **dadurch gekennzeichnet**, daß der Katheter im Falle eines Kunststoffschlauchs (2) am vorderen Ende (3) verschlossen ist.

3. Verwendung nach Anspruch 1, **dadurch gekennzeichnet**, daß der Kunststoffschlauch (2) oder Kunststoffstrang des Katheters am vorderen Ende mit einem Meßkopf versehen ist.

4. Verwendung nach Anspruch 1 oder 3, **dadurch gekennzeichnet**, daß der Kunststoffschlauch (2) oder Kunststoffstrang des Katheters am vorderen Ende mit einer Einrichtung zur spezifischen Organreizung versehen ist.

5. Verwendung nach einem der vorstehenden Ansprüche, **dadurch gekennzeichnet**, daß der Kunststoffschlauch (2) oder Kunststoffstrang des Katheters am vorderen Ende mit einer Einrichtung zur Fixierung des Katheters an der Innenwand des Organs, z. B. durch Ansaugen, versehen ist.

6. Verwendung nach einem der vorstehenden Ansprüche, **dadurch gekennzeichnet**, daß der Kunststoffschlauch (2) oder Kunststoffstrang des Katheters flexibel ist.

7. Meßeinrichtung zur Bestimmung von Motilität und/oder Peristaltik in schlauchförmigen, ihren Inhalt transportierenden Organen mit einem Katheter in Form eines Schlauchs (2) oder Strangs aus einem elektrisch nicht leitenden Kunststoff, der im Meßbereich eine Mehrzahl in definiertem gegenseitigem Abstand angeordnete, den Kunststoffschlauch (2) oder Kunststoffstrang wenigstens teilweise ringförmig umgebende Elektroden (4) trägt, deren Signalzuleitungen oder -ableitungen (7) im Inneren des Schlauchs (2) oder Strangs verlaufen bzw. in diesen eingebettet sind, **gekennzeichnet durch** einen mit den Signalableitungen (7) vom Katheter verbundenen Mehrkanal-Impedanz-Spannungswandler (12), dessen Signalausgänge (13) auf ein Mehrkanalanzeige- oder -schreibgerät (14) und/oder eine Signalerfassungseinrichtung (17) geschaltet sind.

8. Meßeinrichtung nach Anspruch 7, **dadurch gekennzeichnet**, daß der Kunststoffschlauch (2) oder Kunststoffstrang im Meßbereich im wesentlichen starr ist.

9. Meßeinrichtung nach Anspruch 8, **dadurch gekennzeichnet**, daß der Kunststoffschlauch (2) oder Kunststoffstrang im Meßbereich Keramikmaterial enthält.

10. Meßeinrichtung nach Anspruch 8, **dadurch gekennzeichnet**, daß der Kunststoffschlauch (2) in eine Mehrzahl von axialen Längskanälen (6) unterteilt ist, in deren einem die Signalzuleitungen bzw. -ableitungen der Elektroden verlaufen, während die anderen Längskanäle in Öffnungen (5) der äußeren Schlauchwand oder des Katheter-Meßkops münden.

11. Meßeinrichtung nach Anspruch 10, **dadurch gekennzeichnet**, daß die Öffnungen (5) in der Schlauchwand in Längsrichtung des Schlauchs (2) definierte gegenseitige Abstände aufweisen und vorzugsweise im Bereich zwischen zwei Elektroden (4) angeordnet sind.

12. Meßeinrichtung nach Anspruch 8, **dadurch gekennzeichnet**, daß die ringförmigen Elektroden (4) aus einem elektrisch gut leitenden Bandmaterial bestehen und/oder zusätzlich auf der Außenseite eine langzeitstabile Oberflächenschicht mit hoher elektrolytischer Leitfähigkeit und geringer Polarisationsspannung aufweisen.

13. Meßeinrichtung nach Anspruch 8, **dadurch gekennzeichnet**, daß die Elektroden (4) unterschiedliche Breite in Längsrichtung des Kunststoffschlauchs (2) bzw. Kunststoffstrangs aufweisen.

14. Meßeinrichtung nach Anspruch 8, **dadurch gekennzeichnet**, daß der gegenseitige Abstand der Elektroden (4) etwa gleich ist.

15. Meßeinrichtung nach Anspruch 8, **dadurch gekennzeichnet**, daß der gegenseitige Abstand der Elektroden (4) wenigstens in einem Teilabschnitt anders ist als in einem anderen Teilabschnitt des Kunststoffschlauchs (2) bzw. Kunststoffstrangs.

16. Meßeinrichtung in einer Meßvorrichtung nach Anspruch 7, **gekennzeichnet durch** eine Einrichtung zur gleichzeitigen Aufnahem von elektrischen Biosignalen (EMG, EKG etc.) mittels einzelner Bioverstärker (15) mit Signaltrennung.

## Claims

1. Use of a catheter in the form of a tube (2) or cord made of non-electroconductive plastic, which bears in the measuring area a plurality of electrodes (4) arranged at a defined distance from one another and enclosing the plastic tube (2) or plastic cord at least partially in annular fashion, the signal leads to or from which electrodes extend in the interior of the tube (2) or cord in order to obtain signals for evaluating motility and/or peristalsis in tubular organs that transport their contents, or in order to determine the dynamic resilience of tubular conveying organs, by means of simultaneous, multiple impedance measurement.

2. Use according to Claim 1, characterized in that the catheter, in the case of a plastic tube (2), is closed at the front end (3).

3. Use according to Claim 1, characterized in that the plastic tube (2) or plastic cord of the catheter is provided at the front end with a probe.

4. Use according to Claim 1 or 3, characterized in that the plastic tube (2) or plastic cord of the catheter is provided at the front end with a device for specific organ stimulation.

5. Use according to one of the preceding claims, characterized in that the plastic tube (2) or plastic cord of the catheter is provided at the front end with a device for fixing the catheter to the inner wall of the organ, for example by means of suction.

6. Use according to one of the preceding claims, characterized in that the plastic tube (2) or plastic cord of the catheter is flexible.

7. Measuring device for determining motility and/or peristalsis in tubular organs that transport their contents, with a catheter in the form of a tube (2) or cord made of non-electroconductive plastic, which bears in the measuring area a plurality of electrodes (4) arranged at a defined distance from one another and enclosing the plastic tube (2) or plastic cord at least partially in annular fashion, the signal leads (7) to or from which electrodes extend in the interior of the tube (2) or cord or are embedded therein, characterized by a multi-channel impedance voltage transformer (12) which is connected to the signal leads (7) from the catheter and the signal outputs (13) of which are connected to a multi-channel display or multi-channel recorder (14) and/or to a signal-detecting device (17).

8. Measuring device according to Claim 7, characterized in that the plastic tube (2) or plastic cord is essentially rigid in the measuring area.

9. Measuring device according to Claim 8, characterized in that the plastic tube (2) or plastic cord contains ceramic material in the measuring area.

10. Measuring device according to Claim 8, characterized in that the plastic tube (2) is divided into a plurality of axial longitudinal channels (6), in one of which the signal leads to and from the electrodes extend, while the other longitudinal channels (5) open into openings (5) of the outer tube wall or of the catheter probe.

11. Measuring device according to Claim 10, characterized in that the openings (5) in the tube wall have defined distances from one another in the longitudinal direction of the tube (2) and are preferably arranged in the region between two electrodes (4).

12. Measuring device according to Claim 8, characterized in that the annular electrodes (4) are made of a strip material with good electroconductivity and/or additionally have on the outside a long-term stable surface coating with high electrolytic conductivity and low polarization voltage.

13. Measuring device according to Claim 8, characterized in that the electrodes (4) have different widths in the longitudinal direction of the plastic tube (2) or plastic cord.

14. Measuring device according to Claim 8, characterized in that the distance of the electrodes (4) from one another is approximately equal.

15. Measuring device according to Claim 8, characterized in that the distance of the electrodes (4) from one another is different in at least one part of the plastic tube (2) or plastic cord from that in another part thereof.

16. Measuring device in a measuring unit according to Claim 7, characterized by a device for the simultaneous reception of electrical biosignals (EMG, ECG etc.) by means of individual bio-amplifiers (15) with signal separation.

## Revendications

1. Utilisation d'un cathéter en forme de tuyau (2) ou cordon en une matière plastique ne conduisant pas l'électricité, qui porte, dans la zone de mesure, une pluralité d'électrodes (4) entourant au moins en partie, de façon annulaire, le tuyau (2) ou cordon en matière plastique, disposées à des distances définies les unes par rapport aux autres, dont les conducteurs d'amenée ou de sortie de signal s'étendent à l'intérieur du tuyau (2) ou cordon pour produire des signaux afin d'évaluer la motilité et/ou le péristaltisme dans des organes en forme de tuyau, véhiculant leur contenu, ou pour déterminer la flexibilité dynamique d'organes transporteurs en forme de tuyau, à l'aide de mesures d'impédance multiples simultanées.

2. Utilisation selon la revendication 1, caractérisée par le fait que le cathéter, dans le cas d'un tuyau (2) en matière plastique, est fermé à son extrémité avant (3).

3. Utilisation selon la revendication 1, caractérisée par le fait que le tuyau (2) ou cordon en matière plastique du cathéter est muni, à son extrémité avant, d'une tête de mesure.

4. Utilisation selon la revendication 1 ou 3, caractérisée par le fait que le tuyau (2) ou cordon en matière plastique du cathéter est muni, à son extrémité avant, d'un dispositif permettant la stimulation spécifique d'un organe.

5. Utilisation selon l'une quelconque des revendications précédentes, caractérisée par le fait que le tuyau (2) ou cordon en matière plastique du cathéter est muni, à son extrémité avant, d'un dispositif permettant de fixer le cathéter à la paroi intérieure de l'organe, par exemple par aspiration.

6. Utilisation selon l'une quelconque des revendications précédentes, caractérisée par le fait que le tuyau (2) ou cordon en matière plastique du cathéter est flexible.

7. Dispositif de mesure permettant de déterminer la motilité et/ou le péristaltisme d'organes en forme de tuyau, véhiculant leur contenu, à l'aide d'un cathéter en forme de tuyau (2) ou cordon en une matière plastique ne conduisant pas l'électricité, qui porte, dans la zone de mesure, une pluralité d'électrodes (4) entourant au moins en partie, de façon annulaire, le tuyau (2) ou cordon en matière plastique, disposées à des distances définies les unes par rapport aux autres, dont les conducteurs d'amenée ou de sortie de signal (7) s'étendent à l'intérieur du tuyau (2) ou cordon ou sont incorporés dans ce dernier, caractérisé par un transformateur impédance-tension multicanaux (12), relié aux conducteurs de sortie de signal (7) et dont les sorties de signal (13) sont reliées à un appareil d'affichage ou d'enregistrement multicanaux (14) et/ou un dispositif d'aquisition de signal (17).

8. Dispositif de mesure selon la revendication 7, caractérisé par le fait que dans la zone de mesure, le tuyau (2) ou cordon en matière plastique est sensiblement rigide.

9. Dispositif de mesure selon la revendication 8, caractérisé par le fait que dans la zone de mesure, le tuyau (2) ou cordon en matière plastique contient un matériau céramique.

10. Dispositif de mesure selon la revendication 8, caractérisé par le fait que le tuyau (2) en matière plastique est subdivisé en une pluralité de canaux longitudinaux axiaux (6), dans l'un desquels s'étendent les conducteurs d'amenée et de sortie de signal des électrodes, tandis que les autres canaux longitudinaux débouchent dans des orifices (5) de la paroi extérieure du tuyau ou de la tête de mesure du cathéter.

11. Dispositif de mesure selon la revendication 10, caractérisé par le fait que les orifices (5) de la paroi du tuyau sont situés à des distances définies réciproques dans le sens longitudinal du tuyau (2) et sont disposés, de préférence, dans la zone comprise entre deux électrodes (4).

12. Dispositif de mesure selon la revendication 8, caractérisé par le fait que les électrodes (4) de forme annulaire sont constituées par un feuillard bon conducteur électrique et/ou présentent en outre, sur leur face extérieure, une couche superficielle ayant une stabilité de longue durée, une conductivité électrolytique élevée et une faible tension de polarisation.

13. Dispositif de mesure selon la revendication 8, caractérisé par le fait que les électrodes (4) présentent des largeurs différentes dans le sens longitudinal du tuyau (2) ou cordon en matière plastique.

14. Dispositif de mesure selon la revendication 8, caractérisé par le fait que les distances entre les électrodes (4) sont à peu près identiques.

15. Dispositif de mesure selon la revendication 8, caractérisé par le fait que les distances entre les électrodes (4) sont différentes, sur au moins un tronçon, des distances sur un autre tronçon du tuyau (2) ou cordon en matière plastique.

16. Dispositif de mesure dans un appareil de mesure, selon la revendication 7, caractérisé par un dispositif assurant une réception simultanée de signaux électriques biologiques (EMG, ECG, etc.) à l'aide de biorenforçateurs (15) individuels avec séparation des signaux.
